# EUROPEAN PATENT SPECIFICATION

(11) **EP 2 548 597 B1**
(45) Date of publication and mention of the grant of the patent: **10.08.2016**
(21) Application number: 11756106.8
(22) Date of filing: 25.02.2011
(51) Int. Cl.: A61M 5/32, A61M 5/31

(54) **SYRINGE NEEDLE CAP**
SPRITZENNADELKAPPE
CAPUCHON D'AIGUILLE DE SERINGUE

(30) Priority: 18.03.2010 JP 2010062998
(43) Date of publication of application: 23.01.2013
(73) Proprietor: Daikyo Seiko, LTD., Tokyo 131-0031 (JP)
(72) Inventor: KAWACHI, Yasushi, Tokyo 131-0031 (JP)
(74) Representative: TBK
(86) International application number: PCT/JP2011/055152
(87) International publication number: WO 2011/114917

(56) References cited:
- JP-A- 10 305 098
- JP-A- 2003 079 727
- JP-B2- 5 032 069
- US-A1- 2002 062 108
- US-B1- 6 485 474

## Description

### Technical Field

The present invention relates to a syringe needle cap which is fitted to the tip section of a syringe in order to protect a syringe needle protruding from the tip section of a prefilled syringe or a non-prefilled syringe.

### Background Art

It is common in medical practice to transfer a medicinal solution filled in a vial or other type of medical container to a syringe having a syringe needle for usage.

On the other hand, a prefilled syringe in which a predetermined amount of a preselected medicinal solution is prefilled is widely used in medical practice because a predetermined amount of a preselected medicinal solution can be precisely dosed without errors even in emergent circumstances.

As this type of prefilled syringe, it is common in medical practice to use a so-called prefilled syringe having a syringe needle in which a syringe needle is attached to the tip section of a barrel in advance. In this connection, it is generally known to use a syringe cap for protecting a syringe needle protruding from the tip section of a prefilled syringe (See Patent Documents 1 and 2).

Here, each of Patent Documents 1 and 2 discloses a needle cap in which a lining member is loaded in the tip section of a casing, wherein the needlepoint of a syringe needle is stuck into the lining member. The casing of the needle cap is formed by a transparent practice material so the syringe needle can be checked visually from the outside.

Patent Document 3 relates to a protection device for a syringe needle comprising an elastic needle cap of general longitudinal direction presenting a closed distal end and an open proximal end, said cap being formed by a lateral wall defining an inner housing intended to receive the distal part of the body of a needle syringe, and by an end wall capable of being pierced through a part of its thickness by the free end of said needle. Said housing comprises, from said proximal end, an opening, a first portion, a second portion intended to house the distal part of the syringe body which bears said needle, and a third portion which narrows from said second portion in the direction of the back of said housing. The device is characterized in that said lateral wall is further provided with an annular bead disposed in said housing between said first and second portions of said housing.

Patent Document 4 discloses a cap of a closure arrangement of a prefilled disposable syringe which is only removed in advance of utilizing the syringe. The disposable syringe has a syringe body and an injection needle integrated in this body. The closure arrangement includes a stiff plastic cap pushed onto the syringe head and includes a lining in the interior thereof which seals the needle tip. In another syringe type without integrated needle, a connecting cone of a conical connection is formed on the syringe body. The closure arrangement includes an elastomeric closure cap (tip-cap) seated on the connecting cone. The closure cap is covered by a stiff plastic cap connected to the syringe body. In both types of disposable syringes, the plastic cap is welded or cemented while forming a desired break location with the syringe body.
Patent Document 1: JP H10-305098 A
Patent Document 2: US 5 980 495 A
Patent Document 3: US 2002/062108 A
Patent Document 4: US 6 485 474 B1

### Disclosure of Invention

### Problems to be resolved by the Invention

In the needle caps disclosed in Patent Documents 1 and 2, it is impossible to check the needlepoint of the syringe needle visually from the outside because the lining member into which the needlepoint of the syringe needle is stuck is not transparent. There is a difficulty with the sealing performance of the inner space for containing the needle (syringe needle) due to a poor adhesion with the head section of the tip section of a prefilled syringe because the casing which is fitted to the head section is formed by a relatively hard practice material.

The present invention has been made in view of these problems in prior art. The object of the preset invention is to provide a syringe needle cap wherein the entire syringe needle including its tip section protruding from the tip section of a prefilled syringe can be checked visually from the outside and the sealing performance of the inner space covering the syringe needle can be improved.

### Means for solving the Problems

In order to resolve the aforementioned problems, a syringe needle cap according to the present invention is constituted as a cap which is fitted to the tip section of a prefilled syringe for protecting a syringe needle protruding from the tip section of the prefilled syringe, wherein an interior member comprising a transparent elastomer is integrated with the inner surface of a cap main body comprising a transparent resin. A sealing section is formed in the cap main body or the interior member, and the sealing section is closely attached to the outer circumference of a head section which is formed at the tip section of the prefilled syringe. A needlepoint receiving section is formed in the interior member, and the tip section of the syringe needle is stuck into the needlepoint receiving section.

In a syringe needle cap according to the present invention, when the syringe needle cap is fitted to the tip section of the prefilled syringe, the tip section of the syringe needle protruding from the tip section of the prefilled syringe is stuck into the needlepoint receiving section which is formed in the interior member of the syringe needle cap. And then, the inner space of the syringe needle cap covering the syringe needle is successfully sealed by closely contacting the outer circumference of the head section with the sealing section which is formed in the cap main body or the interior member of the syringe needle cap, wherein the head section is formed at the tip section of the prefilled syringe.

Here, in a syringe needle cap according to the present invention, the entire syringe needle protruding from the tip section of the prefilled syringe including its tip section can be visually recognized from the outside of the syringe needle cap because the cap main body comprises a transparent resin and the interior member comprises a transparent elastomer. By this structure, it is possible to recognize troubles like incurve or breakage of the syringe needle, a position gap of the tip section and leakage of the injection solution, and prevent falsification of the prefilled syringe by checking the number of needle sticking to the needlepoint receiving section of the interior member from the needle sticking marks of the injection needle.

In a syringe needle cap according to the present invention, it is preferable to form a circular recess on the tip surface of the cap main body, wherein the center of the circular recess corresponds to the shaft center of the needlepoint receiving section of the interior member because the position gap of the tip section of the injection needle can be checked visually through the circular recess.

Additionally, in a syringe needle cap according to the present invention, it is preferable to use a cap main body having an oxygen passing rate (cm³/m²· day· atm) of 500 or higher and an interior member having an oxygen passing rate (cm³/m²· day atm: at normal temperature, 1mm sample thickness) in a range between 1000 and 40000, because the entire injection needle including its tip section can be certainly sterilized by EOG(ethylene oxide gas) or hydrogen peroxide gas in a condition that the syringe needle cap is fitted to the tip section of the prefilled syringe, that is to say, the tip section of the injection needle is stuck into the needlepoint receiving section of the interior member and the inner space of the syringe needle cap covering the injection needle is sealed.

Furthermore, in a syringe needle cap according to the present invention, it is preferable to use an interior member having a hardness of a range between 20 and 75 (JIS K 6253 A) because the tip of the injection needle is easily and firmly stuck into the needlepoint receiving section of the interior member, the tip section of the injection needle is not damaged and the outer circumference of the head section of the prefilled syringe is smoothly contacted with and firmly and closely attached to the sealing section of the interior member.

Additionally, in a syringe needle cap according to the present invention, it is preferable to set the visible light passing rate (400nm-800nm) of the entire syringe needle cap comprising the cap main body and the interior member to 20% or higher because the entire injection needle protruding from the tip of the prefilled syringe can be easily and certainly checked visually from the outside.

### Effect of Invention

In the syringe needle cap according to the present invention, when the cap is fitted to the tip section of a prefilled syringe, the tip section of the injection needle protruding from the tip section of the prefilled syringe is stuck into the needlepoint receiving section which is formed in the interior member of the syringe needle cap. And then, the inner space of the syringe needle cap covering the injection needle is sealed by closely attaching the outer circumference of the head section formed at the tip section of the prefilled syringe to the sealing section formed in the cap main body or the interior member of the syringe needle cap.

Here, it is possible to visually recognize the entire injection needle including its tip section from the outside of the syringe needle cap, wherein the injection needle protrudes from the tip section of the prefilled syringe, even in a condition that the cap is fitted to the tip section of the prefilled syringe, because the cap main body comprises a transparent resin and the interior member comprises a transparent elastomer. By this configuration, it is possible to recognize incurve or breakage of the injection needle, a position gap of the tip section, and leakage of injection solution, and furthermore the number of needle sticking of the injection needle to the needlepoint receiving section of the interior member by counting the sticking marks, and thereby prevent falsification of the prefilled syringe.

Therefore, in the syringe needle cap according to the present invention, it is possible to visually recognize the entire injection needle protruding from the tip section of the prefilled syringe including its tip section from the outside of the syringe needle cap in a condition that the cap is fitted to the tip section of the prefilled syringe. Additionally, the inner space of the syringe needle cap covering the injection needle can be certainly sealed.

In the syringe needle cap according to the present invention, if the oxygen passing rate (cm³/m²· day atm) of the cap main body is 500 or higher and the oxygen passing rate (cm³/m²· day· atm: at normal temperature, 1mm sample thickness) of the interior member is in a range between 1000 and 40000, the entire injection needle including its tip section can be certainly sterilized by EOG or hydrogen peroxide gas in a condition that the syringe needle cap is fitted to the tip section of the prefilled syringe, that is to say, the tip of the injection needle is stuck into the needlepoint receiving section of the interior member, and the inner space of the syringe needle cap covering the injection needle is sealed.

### Brief Description of Drawings

Fig. 1 shows a front view of a syringe needle cap according to one embodiment of the present invention together with a prefilled syringe.
Fig. 2 shows a front view of a condition that the syringe needle cap shown in Fig. 1 is fitted to the tip section of the prefilled syringe.
Fig. 3 shows an enlarged longitudinal sectional view which shows a sectional structure of the syringe needle cap shown in Fig. 1.
Fig. 4 shows an enlarged longitudinal sectional view which shows a mid-course condition when the syringe needle cap shown in Fig. 1 is being fitted to the tip section of the prefilled syringe.
Fig. 5 shows an enlarged longitudinal sectional view which shows a condition that the syringe needle cap shown in Fig. 1 is fitted to the tip section of the prefilled syringe.
Fig. 6 shows an enlarged longitudinal sectional view which shows a sectional structure of a syringe needle cap according to another embodiment of the present invention.
Fig. 7 shows an enlarged longitudinal sectional view which shows a condition that the syringe needle cap shown in Fig. 6 is fitted to the tip section of the prefilled syringe.

### Embodiments for Implementing the Invention

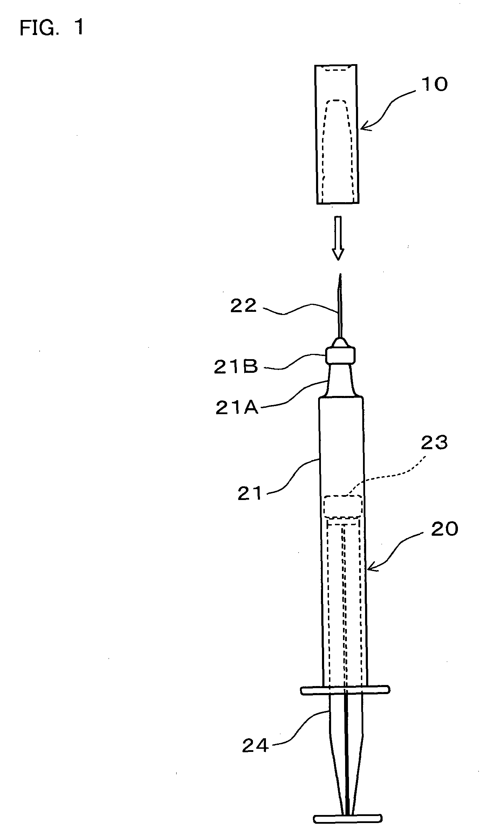

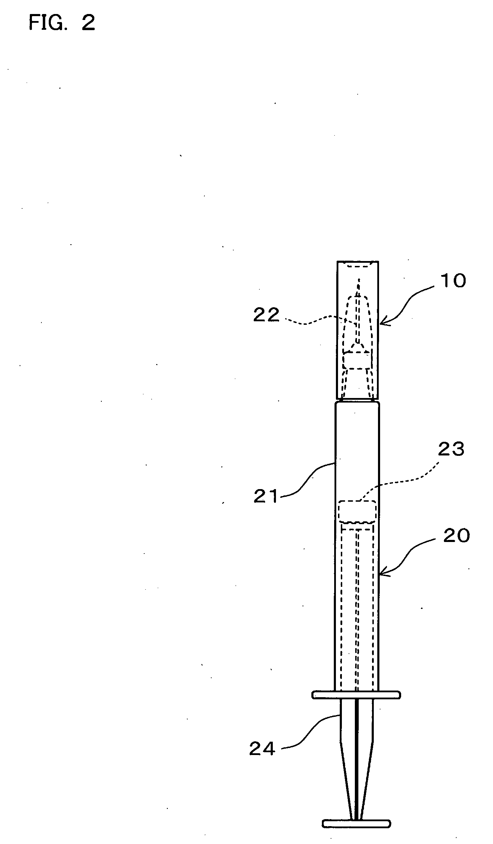

Embodiments of a syringe needle cap according to the present invention will be described referring to the attached drawings. As shown in Figs. 1 and 2, a syringe needle cap 10 according to one embodiment is constituted as a cap which is a fitted to the tip section of a prefilled syringe 20 in which a predetermined amount of a preselected injection solution is filled in advance.

The prefilled syringe 20 is a type of syringe such that an injection needle 22 is coupled and fixed to the tip section of the barrel 21 in advance so that the injection needle 22 protrudes from the barrel 21, the barrel 21 is filled with an injection solution (not shown in drawings), a plunger rod 24 is inserted into the barrel 21, and the tip section of the plunger rod 24 has a gasket 23 for pushing the injection solution out.

A tapered cylindrical neck section 21A and a head section 21B are formed together at the tip section of the barrel 21, the head section 21B continues to the tip section of the neck section 21A and has a slightly larger diameter than the tip of the neck section 21A, and an injection needle 22 is positioned in the center section of the neck section 21A and the head section 21B so that the injection needle 22 protrudes from the head section 21B.

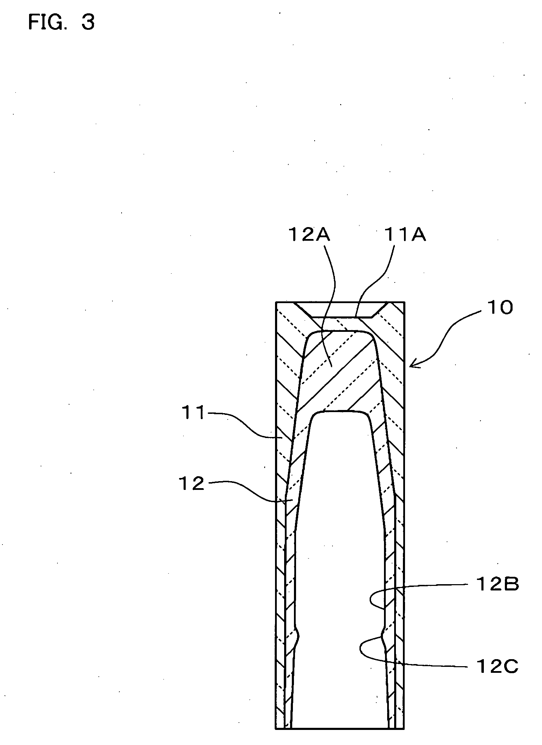

As shown in enlarged views of Figs. 3-5, the syringe needle cap 10 is constituted as a cylindrical cap having a bomb shell shaped inner space and has a two layer structure including an outer layer section 11 as a cap main body and an inner layer section 12 which is integrated with the inside of the outer layer section 11 as an interior member.

Here, the outer layer section 11 as the cap main body is made of a transparent resin, and the inner layer section 12 as the interior member is formed of a transparent elastomer. This kind of two layer structure may have a structure such that the layer section 11 is adhered onto an outer periphery of the inner layer section 12, but in the embodiment of the invention, the inner layer section 12 and the outer layer section 11 are formed together by injection molding through a so-called two-color molding which performs injection molding twice by replacing a part of a mold.

A needlepoint receiving section 12A is formed at the tip section of the inner layer section 12 of the syringe needle cap 10, and the tip section of the injection needle 22 of the prefilled syringe 20 is stuck into the needlepoint receiving section 12A.

A sealing section 12B and a ring shaped bump 12C are formed on the inner periphery of the inner layer section 12 of the syringe needle cap 10, the sealing section 12B is closely attached to the outer circumference of the head section 21B which is formed at the tip section of the barrel 21 of the prefilled syringe 20, and the ring shaped bump 12C is formed for positioning the head section 21B in an axial direction.

On the other hand, a circular recess 11A is formed on the tip section of the outer layer section 11 of the syringe needle cap 10 so as to face the needlepoint receiving section 12A of the inner layer section 12, the center of the circular recess 11A corresponds to the shaft center of the inner layer section 12.

As a transparent elastomer constituting the inner layer section 12 of the syringe needle cap 10, it is suitable to use an elastomer which has an oxygen passing rate in a range between 100 and 40000 (cm³/m^{2·} day atm: at normal temperature, 1mm sample thickness), preferably in a range between 1500 and 30000 (cm³/m^{2·} day· atm: at normal temperature, 1mm sample thickness), more preferably in a range between 1800 and 5000 (cm³/m^{2·} day atm: at normal temperature, 1mm sample thickness), a visible light passing rate (400-800nm) of 20% or higher, preferably 25% or higher, more preferably 30% or higher, a hardness in a range between 20 and 75 (JIS K 6253 A), and a compression permanent distortion (JIS K 6262: 23°Cx24 hours, a miniature test piece) of 27% or smaller, preferably 21% or smaller, more preferably 16% or smaller. For example, olefin elastomer, styrene elastomer (SBS, SIS, SIBS, SEBS, etc.), silicone elastomer (e.g., silicone rubber) are suitable to use.

Examples of the olefin elastomer include α -olefin polymer, α -olefin copolymer, a copolymer of ethylene and unsaturated carboxylic acid, or a copolymer of ethylene and unsaturated carboxylic ester of a carbon number in a range between 2 and 20. For example, olefin elastomer includes ethylene-propylene copolymer, ethylene, 1-butene copolymer, ethylene·1-hexene copolymer, ethylene· 4-methylpentene copolymer, ethylene · 1-octene copolymer, propylene homopolymer, propylene-ethylene copolymer, propylene-ethylene · 1-butene copolymer, 1-butene homopolymer, 1-butene· ethylene copolymer, 1-butene· propylene copolymer, a methylpentene resin, propylene· 1-butene copolymer, ethylene-vinyl acetate copolymer, ethylene· methacrylic acid copolymer, ethylene· methyl methacrylate polymer, etc.

Among them, it is preferable to use a methylpentene resin in view of a better passing rate of oxygen and other gases for the inner layer section 12 of a syringe needle cap. The methylpentene resin may be a homopolymer of a methylpentene compound like 4-methl-1-pentene or 3-methl-1-pentene, or a copolymer including a methylpentene compound as a main monomer. One example of the methylpentene copolymer is a copolymer of a methylpentene compound and α -olefin.

It is preferable to use 4-methyl-1-peritene as a methylpentene compound and use α -olefin of a carbon number in a range between 2 and 24.

Preferable examples of α -olefin of a carbon number in a range between 2 and 24 include ethylene, propylene, 1-butene, 1-pentene, 1-hexene, 1-octene, 1-decene, 1-tetradecene, 1-octadecene, 1-hexadecene, 1-dodecene, 1-tetradodecene, etc. Among them, it is preferable to use 1-hexene, 1-octen, 1-decene, 1-tetradecene, 1-octadecene, 1-hexadecene, 1-dodecene and 1-tetradodecene of a carbon number in a range between 6 and 20.

It is preferable to use a methylpentene copolymer comprising a methylpentene compound of a contained amount of 80% by weight or higher and lower than 100% by weight as a base and α -olefin of a contained amount of 20% by weight or lower and higher than 0% by weight as a copolymerization component in view of better gas passing rate and better oxygen passing rate.

It is preferable to use a methylpentene resin of a melting temperature in a range between 210°C and 280°C, and more preferably in a range between 230°C and 250°C in view of heat resistance.

It is preferable to use a methylpentene resin of a Melt Flow Rate (MFR) in a range between 0.1g/10minutes and 200g/10minutes, more preferably in a range between 1g/ 10minutes and 150g/10minuets under a condition of a temperature of 260°C and a load of 5kg in view of formability and mechanical strength.

A 4-methyl-1-pentene resin is preferable as a methylpentene resin. The 4-methyl-1-pentene resin may be a 4-methyl-1-pentene homopolymer or a 4-methyl-1-pentene· α-olefin copolymer. The base of this copolymer is 4-methyl-1-pentene, and the contained amount is in a range between 80% by weight and 99.9% by weight, preferably in a range between 90% by weight and 99.9% by weight.

Additionally, examples of the α -olefin as a constituent of the copolymer include α -olefin of a carbon number in a range between 2 and 20 like ethylene, propylene, 1-butene, 1-hexen, 1-octen, 1-decene, 1-dodecene, 1-tetradecene, 1-hexadecene, 1-octadecene, 1-eicosene, preferably α -olefin of a carbon number in a range between 6 and 20, and the contained amount is in a rage between 0.1 % by weight and 20% by weight, preferably in a range between 0.1 % by weight and 10% by weight. α -olefin which copolymerizes with 4-metyl-1-pentene can be a single kind or a combination of two kinds or more.

For a 4-methyl-1-penten resin, a marketed product can be used. For example, it may be "Opyuran" (Registered Trademark) made by Mitsui Chemical Corp., TPXMX001, MX002, MX004, MX021, MX321, RT18, DX845 (all of these are Registered Trademarks).

Additionally, it is preferable to use a softener-added methylpentene resin as the inner layer section 12 of the syringe needle cap 10 according to one embodiment. By adding a softener, the tip section of the injection needle 22 can be easily and certainly stuck into the needlepoint receiving section 12A, wherein the injection needle 22 protrudes from the tip of the prefilled syringe 20, and the needlepoint receiving section 12A is formed in the inner layer section 12 of the syringe needle cap 10. It is preferable to use a softener having a viscosity with a low temperature dependence and a high shearing stability and a viscosity in a range between 5 Pa· s and 100 Pa· s.

Examples of the softener for a methylpentene resin include a single kind or two kinds or more of substances selected from a polyvinyl chloride suspension, hydrogenated liquid rubber, chlorine modified liquid rubber, liquid polyolefin, liquid paraffin, vaseline, paraffin, scualane, dioctyl hexane, olive scualane, rice scualane, and higher saturated fatty acid ester.

Examples of a polyvinyl chloride suspension include polyvinyl chloride dispersed in a plasticizing agent like phthalic acid ester, adipic acid ester, etc.

Examples of liquid rubber include liquid butadiene rubber, liquid styrene-butadiene rubber, liquid butadiene-acrylonitrile rubber, liquid chloroprene rubber, polyisobutylene rubber, butyl rubber, liquid isoprene rubber, etc., and a hydride or chlorination product thereof can also be used. As liquid polyolefin, liquid polyolefin oligomer like polyethylene oligomer, polypropylene oligomer, etc. can be used. As higher saturated fatty acid ester, saturated fatty acid ester of a carbon number in a range between 10 and 20 is suitable, and palm oil, castor oil, olive oil, jojoba oil, etc., can be used.

Among them, it is preferable to use a chemical compound which is in a form of liquid at normal temperature like liquid paraffin, liquid polyolefin oligomer, scualane, dioctyl hexane, olive scualane, rice scualane, and more preferably, a chemical compound having a branch structure in a molecule and having less unsaturated bonds like liquid paraffin, liquid polyolefin oligomer, scualane, dioctyl hexane, olive scualane, rice scualane. More preferably, liquid paraffin or liquid polyolefin oligomer can be used. Most preferably, liquid paraffin can be used.

As the liquid polyolefin oligomer, co-oligomer of ethylene and α -olefin which includes no polar group is preferable.

It is preferable to use α -olefin of a carbon number in a range between 2 and 24. As this kind of α -olefin, for example, ethylene, propylene, 1-butene, 1-pentene, 1-hexene, 1-octen, 1-desen, 1-tetradesen, 1-octadesen, 1-hexadesen, 1-dodesen, 1-tetradodesen, etc. can be suitably used.

The amount ratio of the liquid polyolefin oligomer to the methylpentene resin or other transparent elastomer constituting the inner layer section 12 of the syringe needle cap 10 can be arbitrarily adjusted in a range that the injection needle 22 can be easily and firmly stuck into it in the light of the passing rate of oxygen and other gases, the adhesion to a transparent resin constituting the outer layer section 11, and the formability of the inner layer section 12. It is preferable to set the ratio by weight of the liquid polyolefin oligomer to the transparent elastomer in a range between 5:100 and 40:100, more preferably, in a range between 10:100 and 25:100.

The liquid paraffin is a liquid material comprising a mixture of long-chain hydrocarbon chains constituted by alkanes having different lengths, and known materials can be used without limitations within the scope of the present invention.

It is preferable to use liquid paraffin having a kinetic viscosity in a range between 50mm²/s and 90mm²/s at 37.78°C which is measured by a measurement method defined by the Japanese Industrial Standards JIS K2283. The amount ratio of liquid paraffin constituting the inner layer section 12 of the syringe needle cap 10 to the aforementioned methylpentene resin or other transparent elastomer can be arbitrarily adjusted as long as the injection needle 22 can be easily and firmly stuck into the inner layer section 12 in view of the passing rate of oxygen and other gases, the adhesion to the transparent resin constituting the outer layer section 11 and the formability of the inner layer section 12. The weight ratio of the liquid paraffin to the transparent elastomer is set preferably in a range between 5:100 and 40:100, more preferably in a range between 10:100 and 25:100.

Specific examples of the liquid paraffin include white oil made by MORESCO Corp. (for example, P-40, P-70, P-120, P-400) and IP Solvent made by Idemitsu Kosan Co., Ltd. (for example, IP Solvent 2028, IP Solvent 2835). The white oil is a mixture of alkanes having a carbon number in a range between 18 and 30, and the IP solvent is a mixture of alkanes having a carbon number in a range between 16 and 20. For example, because the average molecular number of P-40 is 250, the carbon number is 18. Because the average molecular weight of P-70 is 322, the carbon number is 23. Other examples include "Moresco White P-260", "Moresco White P-350" (kinetic viscosity of 75.5mm²/s at 37.78°C ) made by MORESCO Corp., "Carnation, Klearol, Kaydol, Hydrobrite 1000" made by Sonneborn Corp., "Hicall K-230", "Hicall K-290", "Hicall K-350" made by Kaneda Corp. Additionally, the material is available from Kanto Chemical Co., Inc., Wako Pure Chemical Industries, Ltd., Matsumoto Yushi- Seiyaku Co., Ltd., etc.

It is possible to use liquid paraffin having a carbon number (average carbon number) in a range between 10 and 30, for example.

Additionally it is preferable to use the inner layer section 12 of the syringe needle cap 10 according to one embodiment to which a lubricant agent like silicone oil is added.

It is known that the outer surface and the inner surface of the injection needle are coated by silicone oil for decreasing the puncture resistance in a case of injection to human bodies and easing pains for the puncture, etc. However, all or part of the silicone rubber coated on the injection needle may be torn off when the injection needle is stuck into the syringe needle cap. According to the present invention, because silicone oil included in the inner layer section of the syringe needle cap can be attached to the part of the injection needle where silicone oil was torn off and therefore the aforementioned object can be achieved. It is preferable to set the additive amount of the lubricant agent to a range between 0.2 pts.wt. and 20 pts.wt.

Known materials can be used for the silicone oil. Specific examples include dialkyl silicone oil (for example, dimethylsiloxane, hexamethyldisiloxane, tetramethyldisiloxane, octamethyltrisiloxane, hexamethyltrisiloxane, heptamethyltrisiloxane, decamethyltetrasiloxane, triphloropropylheptamethyltrisiloxane, diethyltetramethyldisiloxane), cyclic dialkyl silicone oil (for example, hexamethylcyclotrisiloxane, hexamethylcyclotrisiloxane, octamethylcyclotetrasiloxane, tetramethylcyclotetrasiloxane, tetra (triphloropropyl) tetramethylcyclotetrasiloxane), methylphenyl silicone oil, polyester modified silicone oil, amino modified silicone oil, alkyl modified silicone oil, alkylaralkyl modified silicone oil, etc.

Additionally, marketed products also can be suitably used. Specific examples of the methylphenyl silicone oil include KF-50-100, KF-50-3000 (made by Shin-etsu Chemical Co., Ltd.), and TSF431 (Toshiba Silicone Corp.). Specific examples of the polyester modified silicone oil include KF-6011 (made by Shin-etsu Chemical Co., Ltd.), and TSF4730 (Toshiba Silicone Corp.).

The oxygen passing rate of the inner layer section 12 was set in a range between 1000 and 40000 (cm³/m²· day· atm: at normal temperature, 1mm sample thickness), because the sterilization speed by gas sterilization becomes significant slow if the oxygen passing rate is lower than 1000, and there is a concern about degradation of medicinal solution by oxygen permeation if the oxygen passing rate is higher than 40000.

The visible light passing rate of the inner layer section 12 is set to 20% or higher because the tip section of the injection needle 22 inside the elastomer becomes invisible if the visible light passing rate is lower than 20%. In the present invention, the higher the visible light passing rate is, the better it is, however, the inventors found that no elastomer material having the visible light passing rate of 70% or higher (at 800nm) has been developed.

The visible light passing rate herein referred to means the lowest passing rate in the visible region (400nm-800nm) unless otherwise described, and it usually means the light passing rate at 400nm because in general the shorter the wavelength is, the lower the light passing rate is. It is desirable to have a light passing rate at 800nm of 50% or higher, preferably 55% or higher, and more preferably 60% or higher. The reason is the same as the aforementioned reason. Additionally, it is preferable to have no absorption peak between 400nm and 800nm in a light passing rate chart, and the light passing rate transits linearly in this range. It is because a clear and colorless material is preferable for visual recognition of the injection needle 22.

A measurement of visible light passing rate was done on a plate sample of 1mm thickness by using a spectral photometer (UV-Vis Spectrophotometer UV-2450: made by Shimadzu Corporation). A laminated body was made by stacking up plate samples, each has 1mm thickness and the visible light passing rate was measured similarly.

The hardness of the inner layer section 12 was set in a range between 20 and 75 (JIS K 6253 A) because the adhesive strength of the syringe needle cap 10 with the tip section of the prefilled syringe 20 becomes weak when the hardness is lower than 20, and it becomes difficult to seal firmly the inner space of the syringe needle cap 10, and the tip section of the injection needle 22 may be damaged or may cause coring if the hardness is higher than 75. A preferable hardness is in a range between 35 and 65, a more preferable hardness is in a range between 40 and 60.

The compression permanent distortion of the inner layer section 12 is set to be smaller than 27% because the syringe needle cap 10 may cause plastic deformation during a long storage, the adhesive strength between the syringe needle cap 10 and the tip section of the prefilled syringe 20 becomes weak, and the inner space of the syringe needle cap 10 may become unable to be firmly sealed, if the compression permanent distortion is higher than 27%. In the present invention, the smaller the compression permanent distortion is, the better it is. However, the inventors have found no elastomer material having a compression permanent distortion smaller than 8% was developed.

On the other hand, as a transparent resin constituting the outer layer 11 of the syringe needle cap 10, it is suitable to use a transparent resin which has an oxygen passing rate (cm³/m²· day atm: at normal temperature, 1 mm sample thickness) of 500 or higher and the visible light passing rate is 70% or higher, for example, methylpentene resin, propylene resin, ethylene resin, polytetrafluoroethylene, polycarbonate, polyethyleneterephthalate, and a mixture of the foregoing material and a material having a good oxygen passing rate.

Among them, it is preferable for the outer layer section 11 to use a methylpentene resin in view of a better permeability of oxygen and other gasses. The methylpentene resin may be the same one which can be used for the inner layer section 12.

In the present invention, it is preferable to use a similar material for both the transparent resin constituting the outer layer section 11 and the transparent elastomer constituting the inner layer section 12, and it is preferable to use a methylpentene resin for both the outer layer section 11 and the inner layer section 12. By using a similar material, the adhesion between the outer layer section 11 and the inner layer section 12 becomes better.

The oxygen passing rate (cm³/m²· day· atm: at normal temperature, 1 mm sample thickness) of the outer layer section 11 is set to 500 or higher because the sterilization speed by gas sterilization becomes significantly slow if the oxygen passing rate is lower than 500. The higher the oxygen passing rate is, the better it is, however, the inventors have found no hard plastic material having an oxygen passing rate of higher than 5000 was developed.

The visible light passing rate of the outer layer section 11 is set to 70% or higher because it becomes impossible to visually recognize the tip section of the injection needle 22 if the visible light passing rate is lower than 70%. In the present invention, the higher the visible light passing rate is, the better it is. Polymethylpentene having an oxygen passing rate of 94% is considered as a plastic material having the highest oxygen passing rate.

As shown in Figs. 1 and 2, a syringe needle cap 10 according to one embodiment configured as aforementioned is pushed and fitted to the tip section of the barrel 21 of the prefilled syringe 20 for protecting the injection needle 22 protruding from the tip section of the prefilled syringe 20.

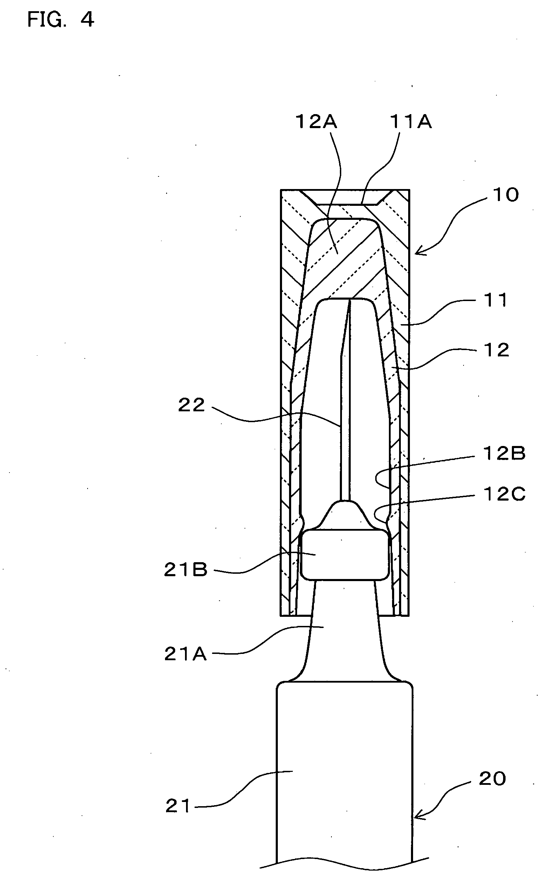

At this time, in a mid-course condition shown in Fig. 4, the head section 21B is temporary positioned to a predetermined position by engaging the upper flange section of the head section 21B to the ring shaped bump 12C, wherein the head section 21B is formed together with the tip section of the barrel 21 and the ring shaped bump 12C is formed in the inner layer section 12 of the syringe needle cap 10. That is to say, the head section 21B is temporary positioned at a position immediately before the injection needle 22 is stuck into the needlepoint receiving section 12A of the inner layer section 12 of the syringe needle cap 10.

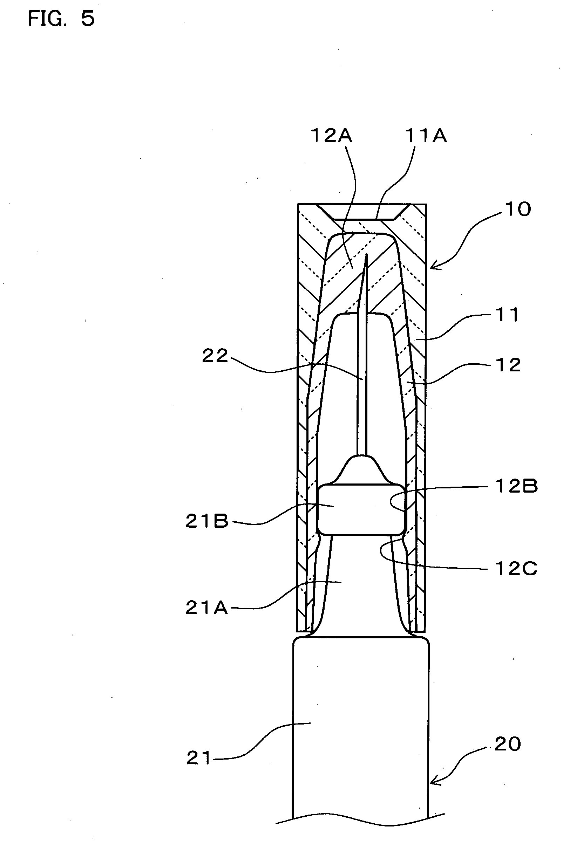

Then, the syringe needle cap 10 is further pushed to the tip section of the barrel 21 while directing the tip section of the injection needle 22 to the center of the syringe needle cap 10. By doing it, as shown in Fig. 5, the tip section of the injection needle 22 is stuck into around the center of the needlepoint receiving section 12A of the inner layer section 12 of the syringe needle cap 10, and the outer circumference of the head section 21B of the tip section of the barrel 21 is closely attached to the sealing section 12B by climbing over the ring shaped bump 12C of the inner layer section 12 of the syringe needle cap 10. At this time, because the hardness of the inner layer section 12 is in a range between 20 and 75, the tip section of the injection needle 22 is easily and firmly stuck into the needlepoint receiving section 12A of the inner layer section 12, and the outer circumference of the head section 21B is closely attached to the sealing section 12B with certainty by smoothly climbing over the ring shaped bump 12C of the inner layer section 12.

By doing this, the injection needle 22 of the prefilled syringe 20 is covered by the syringe needle cap 10, and the inner space of the syringe needle cap 10 covering the injection needle 22 is steadily sealed and therefore the injection needle 22 is maintained in a condition that the injection needle 22 is isolated from the outer air. Here, in the syringe needle cap 10 according to one embodiment, it is possible to visually recognize the entire injection needle 22 protruding from the tip section of the prefilled syringe 20 including its tip section which is stuck into the needlepoint receiving section 12A of the inner layer section 12 because it has a two layer structure including the outer layer section 11 comprising a transparent resin and the inner layer section 12 comprising a transparent elastomer, and the visible light passing rate (400nm-800nm) of the entire outer layer section 11 and inner layer section 12 is set to 20% (400nm) - 50% (800nm) or higher, preferably 25% (400nm) - 55% (800nm) or higher, more preferably 30% (400nm) - 65% (800nm) or higher.

That is to say, it is possible to visually recognize the entire injection needle 22 protruding from the tip section of the prefilled syringe 20 from the outer periphery of the syringe needle cap 10, and it is also possible to visually recognize the position gap of the tip section of the injection needle 22 from the circular recess 11A on the tip surface of the syringe needle cap 10.

Accordingly, in the syringe needle cap 10 according to one embodiment, it is possible to accurately check existence or non-existence of the injection needle 22 protruding from the tip section of the prefilled syringe 20 and troubles like incurve or breakage of the injection needle 22, the position gap of the tip section of the injection needle 22, and leakage of injection solution, and it is also possible to prevent falsification of the prefilled syringe 20 because the number of needle sticking of the injection needle 22 to the needlepoint receiving section 12A of the inner layer section 12 of the syringe needle cap 10 can be recognized by counting the sticking marks.

Additionally, in the syringe needle cap 10 according to one embodiment, it is possible to certainly sterilize the entire injection needle 22 including its tip section using an EOG or hydrogen peroxide gas sterilization apparatus (not shown in drawings) even in a condition shown in Fig. 5, that is to say, in a condition that the tip section of the injection needle 22 is stuck into the needlepoint receiving section 12A of the inner layer section 12, and the inner space of the syringe needle cap 10 covering the injection needle 22 is sealed, because the oxygen passing rate of the outer layer section 11 is set to 500 or higher (cm³/m²· day· atm: at normal temperature, 1mm sample thickness), and the oxygen passing rate of the inner layer section 12 is in a range between 1000 and 40000 (cm³/m²· day atm: at normal temperature, 1mm sample thickness).

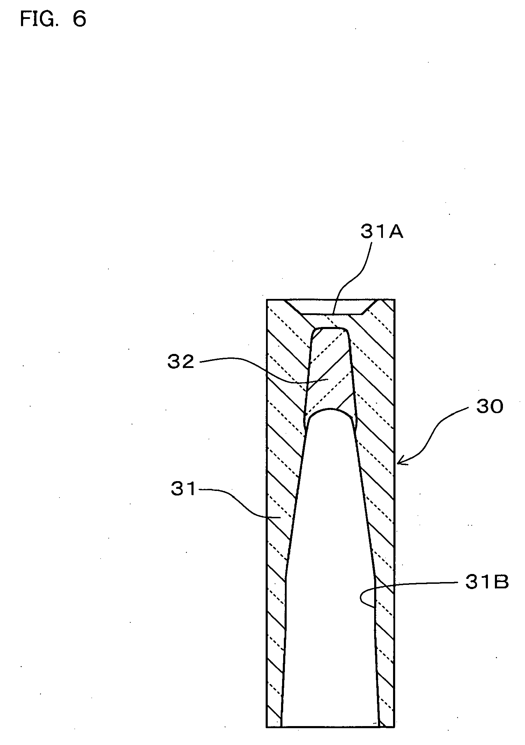

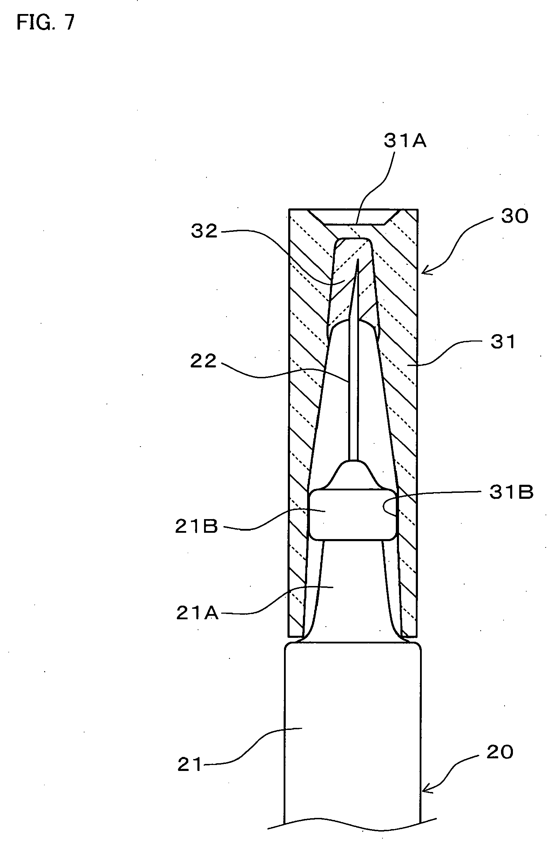

A syringe needle cap according to the present invention is not limited to the syringe needle cap 10 shown in Figs. 3-5, it also can be constituted as a syringe needle cap 30 shown in Figs. 6 and 7, for example. The syringe needle cap 30 is constituted by forming a cap main body 31 having a bomb shell shaped inner space and an interior member 32 which is filled in the inner tip section of the cap main body 31 to form an integrated form.

The cap main body 31 corresponds to the outer layer section 11 of the syringe needle cap 10 shown in Fig. 3 and Fig. 4, and the cap main body 31 comprises a transparent resin which is similar to that used for the outer layer section 11. On the other hand, the interior member 32 corresponds to the needlepoint receiving section 12A of the inner layer section 12 of the syringe needle cap 10 shown in Fig. 3 and Fig. 4, and the interior member 32 comprises a transparent elastomer which is similar to that used for the inner layer section 12.

Here, at the tip of the cap main body 31, a circular recess 31A is formed in the center of the interior member 32 as a needlepoint receiving section to face the interior member 32. A sealing section 31B is formed on the inner periphery of the cap main body 31 to closely attach to the outer periphery of the head section 21B which is formed at the tip section of the barrel 21 of the prefilled syringe 20.

In the syringe needle cap 30 shown in Fig. 6, in a condition that the syringe needle cap 30 is fitted to the tip section of the prefilled syringe 20 as shown in Fig. 7, the tip section of the injection needle 22 is stuck into around the center of the interior member 32 as a needlepoint receiving section, and the outer circumference of the head section 21B of the tip section of the barrel 21 is closely attached to the sealing section 31B of the inner circumference of the cap main body 31. Accordingly, the syringe needle cap 30 shown in Fig. 6 and Fig. 7 achieves an effect which is similar to that of the syringe needle cap 10 shown in Figs. 3-5.

A syringe needle cap according to the present invention is not limited to the syringe needle cap 10 shown in Figs. 3-5 nor the syringe needle cap 30 shown in Figs 6 and 7. For example, it is possible to form a finger grip on the outer circumference of the syringe needle cap 30 shown in Fig. 7, wherein the finger grip has a suitable shape to have an anti-slip function when the syringe needle cap 30 is pulled out from the tip section of the barrel 21 of the prefilled syringe 20, for example, a ring shaped concavo-convex section or a knurl shaped finger grip.

Accordingly, it is possible to form a shallow circular cone shaped recess on the tip surface of the outer layer section 11 of the syringe needle cap 10 shown in Figs. 3-5 in place of the circular recess 11A. Similarly, it is possible to form a shallow circular cone shaped recess on the tip surface of the cap main body 31 of the syringe needle cap 30 shown in Figs. 6 and 7 in place of the circular recess 31A. In these cases, the position gap of the tip section of the injection needle 22 can be easily checked as a position gap from the apex of the circular cone.

### Explanation of the reference numbers

10: syringe needle cap
11: outer layer section
11A: circular recess
12: inner layer section
12A: needlepoint receiving section
12B: sealing section
12C: ring shaped bump
20: prefilled syringe
21: barrel
21A: neck section
21B: head section
22: injection needle
23: gasket
24: plunger rod
30: syringe needle cap
31: cap main body
31A: circular recess
31B: sealing section
32: interior member

## Claims

1. A syringe needle cap (10; 30) which is fitted to a tip section of a prefilled syringe (20) for protecting an injection needle (22) protruding from the tip section of the prefilled syringe (20), wherein
an interior member (12; 32) is integrally formed on an inner surface of a cap main body (11; 31) comprising a transparent resin;
a sealing section (12B; 31B) is formed in the cap main body (31) or the interior member (12), the sealing section (12B; 31B) being formed so as to be closely attached to an outer circumference of a head section (21B) formed at the tip section of the prefilled syringe (20); and
a needlepoint receiving section (12A) is formed in the interior member (12; 32) into which the tip section of the injection needle (22) is to be stuck,
**characterized in that**
the interior member (12; 32) comprises a transparent elastomer.

2. A syringe needle cap (10; 30) according to Claim 1, wherein a circular recess (11A, 31A) is formed on a tip surface of the cap main body (11; 31), a circular recess (11A, 31A) has a center which corresponds to a shaft center of the needlepoint receiving section (12A) of the interior member (12; 32).

3. A syringe needle cap (10; 30) according to Claim 1 or 2, wherein an oxygen passing rate (cm³/m²· day· atm) of the cap main body (11; 31) is 500 or higher and an oxygen passing rate (cm³/m²· day· atm: at normal temperature, 1mm sample thickness) of the interior member (12; 32) is in a range between 10; 3000 and 40000.

4. A syringe needle cap (10; 30) according to either one of Claims 1 through 3, wherein the hardness of the interior member (12; 32) is in a range between 20 and 75 (JIS K 6253 A).

5. A syringe needle cap (10; 30) according to either one of Claims 1 through 4, wherein a visible light passing rate (400nm-800nm) of the entire cap main body (11; 31) and interior member (12; 32) is 20% or higher.

## Patentansprüche

1. Spritzennadelkappe (10; 30), die auf einen Spitzenabschnitt einer vorgefüllten Spritze (20) zum Schützen einer Injektionsnadel (22) gepasst ist, welche von dem Spitzenabschnitt der vorgefüllten Spritze (20) vorragt, wobei
ein Innenbauteil (12; 32) einstückig an einer Innenfläche eines Kappenhauptkörpers (11; 31) ausgebildet ist, der ein transparentes Harz aufweist;
ein Dichtabschnitt (12B; 31B) in dem Kappenhauptkörper (31) oder dem Innenbauteil (12) ausgebildet ist, wobei der Dichtabschnitt (12B; 31B) ausgebildet ist, um eng an einen Außenumfang eines Kopfabschnitts (21B) angebracht zu werden, der an dem Spitzenabschnitt der vorgefüllten Spritze (20) ausgebildet ist; und
ein Nadelpunktaufnahmeabschnitt (12A) in dem Innenbauteil (12; 32) ausgebildet ist, in den der Spitzenabschnitt der Injektionsnadel (22) gesteckt werden soll,
**dadurch gekennzeichnet, dass**
das Innenbauteil (12; 32) ein transparentes Elastomer aufweist.

2. Spritzennadelkappe (10; 30) nach Anspruch 1, wobei eine kreisförmige Vertiefung (11A, 31A) an einer Spitzenfläche des Kappenhauptkörpers (11; 31) ausgebildet ist, eine kreisförmige Vertiefung (11A, 31A) eine Mitte hat, die einer Schaftmitte des Nadelpunktaufnahmeabschnitts (12A) des Innenbauteils (12; 32) entspricht.

3. Spritzennadelkappe (10; 30) nach Anspruch 1 oder 2, wobei eine Sauerstoffdurchlassrate (cm³/m²·Tag·Atm) des Kappenhauptkörpers (11; 31) 500 oder höher und eine Sauerstoffdurchlassrate (cm³/m²·Tag·Atm: bei normaler Temperatur, 1 mm Probendicke) des Innenbauteils (12; 32) in einem Bereich zwischen 10; 3000 und 40000 ist.

4. Spritzennadelkappe (10; 30) nach einem der Ansprüche 1 bis 3, wobei die Härte des Innenbauteils (12; 32) in einem Bereich zwischen 20 und 75 (JIS K 6253 A) ist.

5. Spritzennadelkappe (10; 30) nach einem der Ansprüche 1 bis 4, wobei eine Durchlassrate des sichtbaren Lichts (400 nm bis 800 nm) des gesamten Kappenhauptkörpers (11; 31) und Innenbauteils (12; 32) 20% oder höher ist.

## Revendications

1. Capuchon d'aiguille de seringue (10 ; 30) qui est monté sur une section de pointe d'une seringue pré-remplie (20) pour protéger une aiguille d'injection (22) faisant saillie de la section de pointe de la seringue pré-remplie (20), dans lequel
un élément intérieur (12 ; 32) est formé de manière solidaire sur une surface interne d'un corps principal de capuchon (11 ; 31) comprenant une résine transparente ;
une section d'étanchéité (12B ; 31B) est formée dans le corps principal de capuchon (31) ou l'élément intérieur (12), la section d'étanchéité (12B ; 31B) étant formée afin d'être étroitement fixée à une circonférence externe d'une section de tête (21B) formée au niveau de la section de pointe de la seringue pré-remplie (20) ; et
une section de réception de pointe d'aiguille (12A) est formée dans l'élément intérieur (12 ; 32) dans laquelle la section de pointe de l'aiguille d'injection (22) doit être enfoncée,
**caractérisé en ce que**
l'élément intérieur (12 ; 32) comprend un élastomère transparent.

2. Capuchon d'aiguille de seringue (10 ; 30) selon la revendication 1, dans lequel un évidement circulaire (11A, 31A) est formé sur une surface de pointe du corps principal de capuchon (11 ; 31), un évidement circulaire (11A, 31A) a un centre qui correspond à un centre de tige de la section de réception de pointe d'aiguille (12A) de l'élément intérieur (12 ; 32).

3. Capuchon d'aiguille de seringue (10 ; 30) selon la revendication 1 ou 2, dans lequel un débit de passage d'oxygène (cm³/m²·jour.atm) du corps principal de capuchon (11 ; 31) est de 500 ou supérieur et un débit de passage d'oxygène (cm³/m².jour.atm : à température ambiante, épaisseur d'échantillon 1 mm) de l'élément intérieur (12 ; 32) est dans une plage comprise entre 10 ; 3 000 et 40 000.

4. Capuchon d'aiguille de seringue (10 ; 30) selon l'une des revendications 1 à 3, dans lequel la dureté de l'élément intérieur (12 ; 32) est dans une plage comprise entre 20 et 75 (JIS K 6253 A).

5. Capuchon d'aiguille de seringue (10 ; 30) selon l'une des revendications 1 à 4, dans lequel une vitesse de passage de lumière visible (400 nm-800 nm) de tout le corps principal de capuchon (11 ; 31) et de l'élément intérieur (12 ; 32) est de 20 % ou supérieure.
